(19) Europäisches Patentamt
European Patent Office
Office européen des brevets

(11) Veröffentlichungsnummer: **0 266 532**
**A1**

(12) **EUROPÄISCHE PATENTANMELDUNG**

(21) Anmeldenummer: **87113797.2**

(22) Anmeldetag: **22.09.87**

(51) Int. Cl.4: **C07D 233/56**, C07D 233/84, C07D 233/70, C07D 233/22, C07D 233/20, C07D 233/38, C07D 233/42, A61K 31/415

(30) Priorität: **03.10.86 CH 3955/86**

(43) Veröffentlichungstag der Anmeldung:
**11.05.88 Patentblatt 88/19**

(84) Benannte Vertragsstaaten:
**AT BE CH DE FR GB IT LI NL**

(71) Anmelder: **F. HOFFMANN-LA ROCHE & CO. Aktiengesellschaft**

**CH-4002 Basel(CH)**

(72) Erfinder: **Link, Helmut, Dr.**
**Dammerkirchstrasse 70**
**CH-4056 Basel(CH)**

(74) Vertreter: **Notegen, Eric-André et al**
**Grenzacherstrasse 124 Postfach 3255**
**CH-4002 Basel(CH)**

(54) **1,3-Disubstituierte Imidazoliumsalze.**

(57) Die den neuen Imidazoliumhydrogencarbonaten der allgemeinen Formel

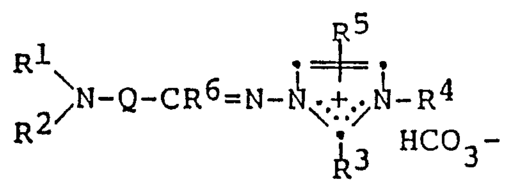

I

worin das Symbol Q Arylen oder Heteroarylen, die Gruppe $-NR^1R^2$ eine basische Aminogruppe, $R^3$ Wasserstoff, niederes Alkyl, niederes Hydroxyalkyl, niederes Alkoxyalkyl, niederes Halogenalkyl, niederes Alkylthio, niederes Alkoxy oder die Gruppe $-(A)_n-Ra$, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, $R^5$ Wasserstoff, niederes Alkyl, niederes Hydroxyalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl-oder basische Aminogruppe, A Vinylen oder niederes Alkylen, n die Zahl 0 oder 1 und die gestrichelte Linie eine zusätzliche Doppelbindung bedeuten, entsprechenden pharmazeutisch annehmbaren Salze besitzen wertvolle antibakterielle, antimykotische, protozoozide und/oder anthelmintische Eigenschaften. Die Imidazoliumbicarbonate der Formel I können verwendet werden, um derartige pharmazeutisch annehmbare, insbesondere wasserlösliche Salze auf einfache und billige Weise herzustellen.



Xerox Copy Centre

## 1,3-Disubstituierte Imidazoliumsalze

Die vorliegende Erfindung betrifft Imidazoliumhydrogencarbonate der allgemeinen Formel

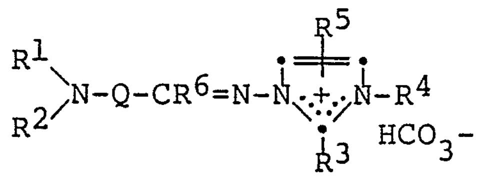

I

worin das Symbol Q Arylen oder Heteroarylen, die Gruppe $-NR^1R^2$ eine basische Aminogruppe, $R^3$ Wasserstoff, niederes Alkyl, niederes Hydroxylalkyl, niederes Alkoxyalkyl, niederes Halogenalkyl, niederes Alkylthio, niederes Alkoxy oder die Gruppe $-(A)_n$ -Ra, $R^4$ eine gesättigte oder partiell ungesättigte niedere Kohlenwasserstoffgruppe, eine basische Aminogruppe oder die Gruppe -N = CRc-Rb, -CHRcRd, -NH-CHRcRd, -NH-CO-Re oder -CHRc-CO-Re, $R^5$ Wasserstoff, niederes Alkyl, niederes Hydroxyalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl-oder basische Aminogruppe, A Vinylen oder niederes Alkylen, n die Zahl 0 oder 1 und die gestrichelte Linie eine zusätzliche Doppelbindung ledeuten.
Die diesen neuen Verbindungen entsprechenden pharmazeutisch annehmbaren Salze besitzen wertvolle pharmakologische Eigenschaften. Sie besitzen insbesondere antibakterielle, antimykotische, protozoozide und/oder anthelmintische Eigenschaften. Die obigen Verbindungen der Formel I können verwendet werden, um derartige pharmazeutisch annehmbare, insbesondere wasserlösliche Salze auf einfache und billige Weise herzustellen.

Gegenstand der vorliegenden Erfindung sind: Die obigen Verbindungen der Formel I als solche; ein Verfahren zu deren Herstellung; ein Verfahren zur Herstellung der diesen entsprechenden pharmazeutisch annehmbaren Salze; sowie die Verwendung dieser Verbindungen zur Herstellung der diesen entsprechenden pharmazeutisch annehmbaren Salze.

Der Ausdruck "nieder" bezeichnet Reste und Verbindungen mit höchstens 7, vorzugsweise höchstens 4 Kohlenstoffatomen. Der Ausdruck "Alkyl" für sich allein genommen oder in Zusammensetzungen, wie "Alkylgruppen", "Alkoxy" und "Alkylthio", bezeichnet geradkettige oder verzweigte, gesättigte Kohlenwasserstoffreste, wie Methyl, Aethyl, n-Propyl, i-Propyl, n-Butyl, s-Butyl, i-Butyl und t-Butyl.

Der Ausdruck "gesättigte oder partiell ungesättigte Kohlenwasserstoffgruppe" bezeichnet offenkettige und cyclische Gruppen und Kombinationen davon. Beispiele für gesättigte und partiell ungesättigte niedere Kohlenwasserstoffgruppen sind: Niedere Alkylgruppen, wie Methyl, Aethyl, Propyl, i-Propyl, s-Butyl und i-Butyl; niedere Alkenylgrup pen, wie 2-Propenyl, 2-Butenyl, 3-Butenyl und 2-Methyl-2-propenyl; gegebenenfalls durch niedere Alkylgruppen substituierte niedere Cycloalkylgruppen, wie Cyclopropyl, Cyclopentyl, 2-Methylcyclopentyl, Cyclohexyl und 3-Methyl-cyclohexyl; gegebenenfalls durch niedere Alkylgruppen substituierte niedere Cycloalkenylgruppen, wie 3-Cyclopentenyl, 1-Methyl-3-cyclopentenyl und 3-Cyclohexenyl; durch niedere Cycloalkyl-oder Cycloalkenylgruppen substituierte niedere Alkyl-oder Alkenylgruppen, wie Cyclopropylmethyl, Cyclopropyläthyl, Cyclopentylmethyl, Cyclohexylmethyl, 2-Cyclohexenylmethyl und 3-Cyclopropyl-2-propenyl. Die bevorzugten niederen Kohlenwasserstoffgruppen sind gesättigt. Die niederen Alkyl-und niederen Cycloalkylgruppen, insbesondere die niederen Alkylgruppen, sind besonders bevorzugte niedere Kohlenwasserstoffgruppen.

Der Ausdruck "Aryl" bezeichnet carbocyclische aromatische Gruppen, vorzugsweise mono-oder bicyclische Gruppen, d.h. Phenyl-und Naphthylgruppen, insbesondere Phenylgruppen. Diese Gruppen sind gegebenenfalls durch einen, zwei oder drei Substituenten aus der Gruppe bestehend aus basischem Amino, niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und Cyano substituiert. Diese Gruppen sind vorzugsweise unsubstituiert oder durch einen, zwei oder drei Substituenten aus der Gruppe bestehend aus niederem Alkyl, niederem Alkoxy, niederem Alkylthio, Halogen, Nitro und Di(niederem Alkyl)Amino substituiert.

Der Ausdruck "Arylen" bezeichnet carbocyclische aromatische Gruppen mit zwei freien Valenzen, vorzugsweise mono-oder bicyclische Gruppen, d.h. Phenylen-und Naphthylengruppen, insbesondere 1,4-oder 1,2-Phenylengruppen und im speziellen 1,4-Phenylengruppen. Diese Gruppen können durch einen oder zwei Substituenten aus der Gruppe bestehend aus niederem Alkyl, niederem Alkoxy, niederem Alkylthio, niede rem Alkanoyl, niederem Alkoxycarbonyl, Aryl (insbesondere Phenyl), Halogen, Trifluormethyl, Hydroxy, Nitro und Cyano substituiert sein. Sie sind vorzugsweise unsubstituiert.

Der Ausdruck "Heteroaryl" bezeichnet heterocyclische aromatische Gruppen, vorzugsweise mono-oder bicyclische Gruppen, insbesondere gegebenenfalls mit einem Benzolring annellierte 5-oder 6-gliedrige aromatische Heterocyclen, welche gegebenenfalls durch einen, zwei oder drei Substituenten aus der Gruppe bestehend aus basischem Amino, niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und Cyano substituiert sein können. Die 5-gliedrigen aromatischen Heterocyclen enthalten als Heteroringglieder vorzugsweise ein Sauerstoff-oder Schwefelatom oder eine Iminogruppe und gegebenenfalls noch ein oder zwei Stickstoffatome. Die 6-gliedrigen aromatischen Heterocyclen enthalten als Ringglieder vorzugsweise ein, zwei oder drei Stickstoffatome.

Der Ausdruck "Heteroarylen" bezeichnet heterocyclische aromatische Gruppen mit zwei freien Valenzen, vorzugsweise mono-oder bicyclische Gruppen, insbesondere gegebenenfalls mit einem Benzolring annellierte 5-und 6-gliedrige aromatische Heterocyclen mit zwei freien Valenzen, welche noch durch einen oder zwei Substituenten aus der Gruppe bestehend aus niederem Alkyl, niederem Alkoxy, niederem Alkylthio, niederem Alkanoyl, niederem Alkoxycarbonyl, Aryl (insbesondere Phenyl), Halogen, Trifluormethyl, Hydroxy, Nitro und Cyano substituiert sein können.

Der Ausdruck "Halogen" bezeichnet die vier Formen Fluor, Chlor, Brom und Jod.

Der Ausdruck "basische Aminogruppe" oder "basisches Amino" bezeichnet unsubstituierte oder mono-oder disubstituierte Aminogruppen mit basischem Charakter. Die basischen Aminogruppen können durch die allgemeine Formel -NRR′ dargestellt werden. Darin bedeuten vorzugsweise R Wasserstoff oder niederes Alkyl und R′ Wasserstoff oder eine gesättigte oder partiell ungesättigte niedere Kohlenwasserstoffgruppe, welche gegebenenfalls durch eine oder zwei niedere Alkoxy-oder Hydroxygruppen oder durch eine Amino-, niedere Alkylamino-, niedere Dialkylamino-, Oxo-, niedere Alkoxycarbonyl-oder niedere Alkylendioxygruppe substituiert ist, oder R und R′ zusammen mit dem Stickstoffatom einen gegebenenfalls durch eine oder zwei niedere Alkylgruppen substituierten 4-bis 7-gliedrigen gesättigten N-Heterocyclus, der als Ringglied anstelle einer Methylengruppe ein Sauerstoff-oder Schwefelatom oder die Gruppe >SO, >$SO_2$, >CO, >CH-Rf oder >N-Rg enthalten kann, wobei Rf Hydroxy, niederes Alkanoyl, niederes Alkoxycarbonyl, Carbamoyl, mono-oder di(niederes Alkyl)carbamoyl oder eine gegebenenfalls über ein Sauerstoffatom gebundene, gesättigte oder partiell ungesättigte niedere Kohlenwasserstoffgruppe, welche gegebenenfalls noch durch eine oder zwei niedere Alkoxy-oder Hydroxygruppen oder durch eine Amino-, niedere Alkylamino-, niedere Dialkylamino-, Oxo-, niedere Alkoxycarbonyl-oder niedere Alkylendioxygruppe substituiert ist, und Rg Wasserstoff, niederes Alkanoyl, niederes Alkoxycarbonyl, Carbamoyl, mono-oder di(niederes Alkyl)-carbamoyl oder eine gesättigte oder partiell ungesättigte niedere Kohlenwasserstoffgruppe, welche gegebenenfalls durch eine oder zwei niedere Alkoxy-oder Hydroxygruppen oder durch eine Amino-, niedere Alkylamino-, niedere Dialkylamino-, Oxo-, niedere Alkoxycarbonyl-oder niedere Alkylendioxygruppe substituiert ist, bedeuten.

Besonders bevorzugte basische Aminogruppen -NRR′ sind diejenigen, worin R Wasserstoff oder niederes Alkyl und R′ eine gesättigte niedere Kohlenwasserstoffgruppe oder R und R′ zusammen mit dem Stickstoffatom einen gegebenenfalls durch eine Hydroxy-, niedere Alkoxy-, niedere Hydroxyalkyl-oder niedere Alkoxyalkylgruppe substituierten 4-, 5-oder 6-gliedrigen gesättigten N-Heterocyclus oder einen gegebenenfalls durch eine oder zwei niedere Alkylgruppen substituierten 6-gliedrigen gesättigten N-Heterocyclus, der anstelle einer Methylengruppe ein Sauerstoff-oder Schwefelatom oder eine Imino-oder niedere Alkyliminogruppe enthält, bedeuten.

Ganz besonders bevorzugte basische Aminogruppen -NRR′ sind diejenigen, worin R und R′ je niederes Alkyl oder zusammen mit dem Stickstoffatom 1-Pyrrolidinyl, 1-Piperidinyl, 4-Morpholinyl, 2,6-Dimethyl-4-morpholinyl, 4-Thiomorpholinyl oder 4-Methyl-1-piperazinyl bedeuten.

Enthält eine Verbindung der Formel I mehr als eine basische Aminogruppe, so können diese gleich oder verschieden sein.

Der Ausdruck "Abgangsgruppe" bezeichnet vorzugsweise Halogenatome, wie Chlor, Brom und Jod, niedere Alkylsulfonyloxygruppen, wie Methylsulfonyloxy, und Arylsulfonyloxygruppen, wie p-Tolylsulfonyloxy.

In einer speziellen Ausführungsform betrifft die vorliegende Erfindung Verbindungen der obigen Formel I, worin $R^3$ niederes Alkyl bedeutet.

In einer weiteren speziellen Ausführungsform betrifft die vorliegende Erfindung Verbindungen der obigen Formel I, worin $R^3$ die Gruppen $-(A)_n-Ra$ bedeutet. Vorzugsweise bedeuten entweder n die Zahl 0 und Ra eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe, wobei Ra besonders bevorzugt eine unsubstituierte oder durch niederes Alkyl, niederes Alkoxy oder Halogen mono-substituierte Phenylgruppen, wie Phenyl, p-Chlorphenyl, p-Tolyl und m-Methoxyphenyl, bedeu tet, oder n die Zahl 1, A Vinylen und Ra die Gruppe -NRR', wobei R und R' je Wasserstoff oder niederes Alkyl oder zusammen mit dem Stickstoffatom einen gegebenenfalls durch eine oder zwei niedere Alkylgruppen substituierten, 5-oder 6-gliedrigen gesättigten Heterocyclus, der ein oder zwei Stickstoffatome oder ein Stickstoffatom und ein Sauerstoff-oder Schwefelatom als Heteroatome enthält, bedeuten, und R und R' dabei vorzugsweise je niederes Alkyl, insbesondere Methyl, oder zusammen mit dem Stickstoffatom 4-Morpholinyl oder 1-Piperidinyl bedeuten.

Das Symbol $R^5$ bedeutet vorzugsweise Wasserstoff.

In einer bevorzugten Ausführungsform bedeutet $R^4$ die Gruppe $-N=CR^6-Q-NR^1R^2$.

In einer weiteren bevorzugten Ausführungsform bedeutet $R^4$ die Gruppe -N=CRc-Rb, wobei Rc vorzugsweise Wasserstoff und Rb vorzugsweise eine unsubstituierte oder durch einen, zwei oder drei Substituenten aus der Gruppe bestehend aus niederem Alkyl, niederem Alkoxy, niederem Alkylthio, Halogen und Nitro substituierte Phenylgruppe bedeuten. Ganz besonders bevorzugt bedeutet Rb p-Nitrophenyl.

Das Symbol Q bedeutet vorzugsweise gegebenenfalls durch einen oder zwei Substituenten aus der Gruppe bestehend aus niederem Alkyl und niederem Alkoxy substituiertes 1,4-Phenylen. In einer besonders bevorzugten Ausführungsform bedeutet das Symbol Q 1,4-Phenylen.

$R^1$ und $R^2$ bedeuten vorzugsweise je niederes Alkyl. In einer besonders bevorzugten Ausführungsform bedeuten $R^1$ und $R^2$ je Methyl.

Das Symbol $R^6$ bedeutet vorzugsweise Wasserstoff.

Die nachfolgend aufgeführten Imidazoliumsalze sind bevorzugte Vertreter der durch die allgemeine Formel I definierten Stoffklasse:

1,3-Bis[[p-(dimethylamino)benzyliden]amino]-2-[2-(dimethylamino)vinyl]imidazoliumhydrogencarbonat,
1,3-Bis[[p-(dimethylamino)benzyliden]amino]-2-isopropylimidazoliumhydrogencarbonat,
1,3-Bis[[p-(dimethylamino)benzyliden]amino]-2-methylimidazoliumhydrogencarbonat und
1,3-Bis[[p-(dimethylamino)benzyliden]amino]-2-phenylimidazoliumhydrogencarbonat.

Die neuen Verbindungen der Formel I können erfindungsgemäss hergestellt werden, indem man eine Verbindung der allgemeinen Formel

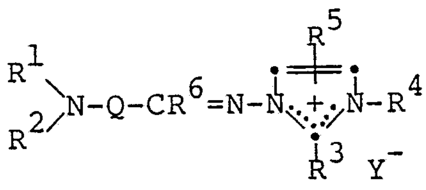

II

worin $R^1$, $R^2$, $R^3$, $R^4$, $R^5$, $R^6$, Q und die gestrichelte Linie obige Bedeutung besitzen, und $Y^-$ ein Anion bedeutet,

mit einer konzentrierten, wässrigen Alkalimetallhydrogencarbonatlösung behandelt.

Vorzugsweise verwendet man dabei eine gesättigte Hydrogencarbonatlösung. Das bevorzugte Alkalimetall ist Natrium. Das erfindungsgemässe Verfahren kann auch in Gegenwart eines organischen Lösungsmittels durchgeführt werden, wobei insbesondere mit Wasser nicht mischbare Lösungsmittel, beispielsweise halogenierte Kohlenwasserstoffe, wie Chloroform, in Frage kommen. $Y^-$ ist vorzugsweise ein Halogenid, insbesondere ein Chlorid, Bromid oder Jodid. Die Reaktiion wird vorzugsweise bei Raumtemperatur durchgeführt.

Die erfindungsgemässen Imidazoliumhydrogencarbonate sind im Reaktionsmedium praktisch unlöslich und können durch Filtration isoliert werden.

Wie bereits erwähnt, können diese Imidazoliumhydrogencarbonate verwendet werden, um auf einfache und billige Weise entsprechende pharmazeutisch annehmbare, und insbesondere wasserlösliche Salze herzustellen. Die Ueberführung der Imidazoliumhydrogencarbonate in die entsprechenden pharmazeutisch annehmbaren Salze der allgemeinen Formel

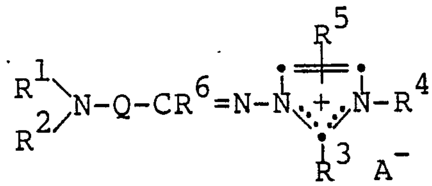

III

worin $R^1$, $R^2$, $R^3$, $R^4$, $R^5$, $R^6$, Q und die gestrichelte Linie obige Bedeutung besitzen, und $A^-$ ein pharmazeutisch annehmbares Anion bedeutet,
kann dadurch bewerkstelligt werden, dass man die Imidazoliumhydrogencarbonate mit der dem gewünschten pharmazeutisch annehmbaren Anion entsprechenden Säure behandelt. Als Lösungsmittel verwendet man vorzugsweise Wasser und niedere Alkohole, wie Methanol, oder Mischungen davon. Die Reaktion wird vorzugsweise bei Raumtemperatur durchgeführt.

Geeignete den pharmazeutisch annehmbaren Anionen entsprechende Säuren sind z.B.: Essigsäure, Benzolsulfonsäure, Benzoesäure, Weinsäure, Bromwasserstoff, Chlorwasserstoff, Citronensäure, Fumarsäure, Apfelsäure, Maleinsäure, Mandelsäure, Methansulfonsäure, Phosphorsäure, Bernsteinsäure, Schwefelsäure und p-Toluolsulfonsäure.

Im Hinblick darauf, dass als Reaktionsprodukt, neben dem gewünschten Salz und Wasser, Kohlendioxid entsteht, das laufend entweicht, brauchen die obigen Säuren nicht in grossem Ueberschuss eingesetzt zu werden. Je nach Säurestärke ist überhaupt kein Ueberschuss erforderlich.

Die Verbindungen der Formel II können hergestellt werden, indem man
a) eine Verbindung der allgemeinen Formel

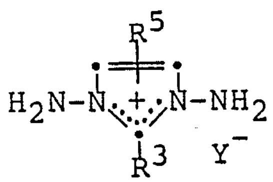

IVa oder

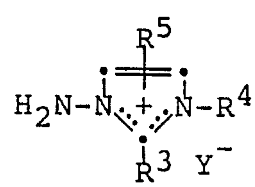

IVb

worin $R^3$, $R^4$, $R^5$, die gestrichelte Linie und $Y^-$ obige Bedeutung besitzen,
mit einer Carbonylverbindung der allgemeinen Formel
$R^1R^2N\text{-}Q\text{-}CO\text{-}R^6$ V
worin $R^1$, $R^2$, $R^6$ und Q obige Bedeutung besitzen, umsetzt, oder
b) eine Verbindung der allgemeinen Formel

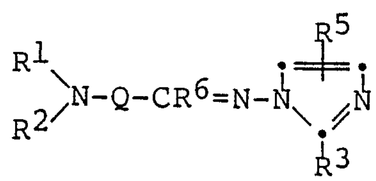

VI

worin $R^1$, $R^2$, $R^3$, $R^5$, $R^6$ und Q obige Bedeutung besitzen,
mit einer Verbindung der allgemeinen Formel
$R^{41}\text{-}X$ VII
worin $R^{41}$ eine gesättigte oder partiell ungesättigte niedere Kohlenwasserstoffgruppe oder die Gruppe -CHRcRd oder -CHRc-CO-Re und X eine Abgangsgruppe bedeuten, und Rc, Rd und Re obige Bedeutung besitzen,
umsetzt, oder
c) eine Verbindung der allgemeinen Formel

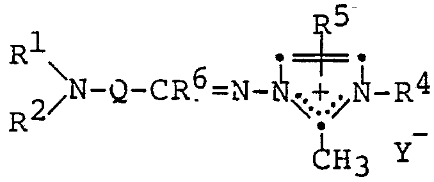

IIa

worin $R^1$, $R^2$, $R^4$, $R^5$, $R^6$, Q, $Y^-$ und die gestrichelte Linie obige Bedeutung besitzen, mit einem Aldehyd der allgemeinen Formel

Ra-CHO VIII

worin Ra obige Bedeutung besitzt, kondensiert, oder

d) eine Verbindung der allgemeinen Formel

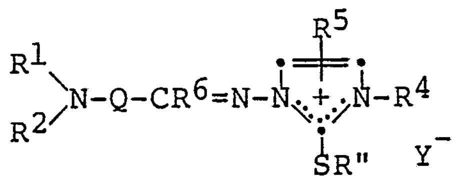

IIb

worin $R^1$, $R^2$, $R^4$, $R^5$, $R^6$, Q, $Y^-$ und die gestrichelte Linie obige Bedeutung besitzen, und R″ niederes Alkyl bedeutet,

mit Ammoniak oder einem primären oder sekundären basischen Amin umsetzt.

Bei verschiedenen der obigen Verfahren a) - d) ist es notwendig, die in den Ausgangsstoffen allfällig vorhandenen reaktionsfähigen Amino-und/oder Hydroxylgruppen durch Schutzgruppen zu blockieren. Diese Fälle sind für den Fachmann ohne weiteres erkennbar, und auch die Auswahl der jeweils geeigneten Schutzgruppen bereitet ihm keine Schwierigkeiten.

Bei der Umsetzung von Aminen mit Aldehyden oder Ketonen gemäss Verfahrensvariante a) handelt es sich um eine an sich bekannte und jedem Fachmann geläufige Reaktion. Als Lösungsmittel eignen sich beispielsweise Wasser, niedere Fettsäuren, wie Essigsäure und Propionsäure, niedere Alkohole, wie Methanol, Aethanol, 1-Propanol und 2-Propanol, niedere Fettsäureester, wie Essigester, niedere Aether, wie Diäthyläther, t-Butylmethyläther, Aethylenglykoldimethyläther, Tetrahydrofuran und Dioxan, halogenierte niedere Kohlenwasserstoffe, wie Methylenchlorid, Chloroform und Aethylenchlorid, aromatische Kohlenwasserstoffe, wie Benzol, Toluol und Xylol, Acetonitril, N,N-Dimethylformamid und Dimethylsulfoxid. Die Reaktionstemperatur ist nicht kritisch. Die Reaktion kann beispielsweise in einem Bereich von etwa 0°C bis zur Siedetemperatur des gewählten Lösungsmittels durchgeführt werden. Man arbeitet jedoch vorzugsweise bei Raumtemperatur. Bei der Umsetzung mit den weniger reaktiven Ketonen der Formel V ($R^6$ = niederes Alkyl) verwendet man vorzugsweise ein Kondensationsmittel, wie Triäthyloxoniumtetrafluoroborat.

Gemäss Verfahrensvariante b) können Verbindungen der Formel II, worin $R^4$ eine gesättigte oder partiell ungesättigte niedere Kohlenwasserstoffgruppe oder die Gruppe -CHRcRd oder -CHRc-CO-Re bedeutet, durch Alkylierung von Imidazolderivaten der Formel VI hergestellt werden. Es handelt sich auch hierbei um eine an sich bekannte und jedem Fachmann geläufige Reaktion. Geeignete Lösungsmittel sind beispielsweise halogenierte niedere Kohlenwasserstoffe, wie Methylenchlorid, Chloroform und Aethylenchlorid, offenkettige oder cyclische Aether, wie Diäthyläther, t-Butylmethyläther, Aethylenglykoldimethyläther, Tetrahydrofuran und Dioxan, niedere Fettsäureester, wie Essigester, niedere Alkohole, wie Methanol, Aethanol, 1-Propanol und 2-Propanol, aromatische Kohlenwasserstoffe, wie Benzol, Toluol und Xylol, Acetonitril, N,N-Dimethylformamid und Dimethylsulfoxid. Man verwendet vorzugsweise einen niederen Alkohol, wie 2-Propanol, oder Acetonitril. Die Reaktionstemperatur ist nicht kritisch. Man kann beispielsweise in einem Bereich von etwa 0°C bis zur Siedetemperatur des Lösungsmittel, vorzugsweise in einem Bereich von etwa Raumtemperatur bis etwa 60°C, arbeiten.

Gemäss Verfahrensvariante c) können Verbindungen der Formel II, worin $R^3$ die Gruppe -CH=CH-Ra bedeutet, durch Kondensation einer Verbindung der Formel IIa mit einem Aldehyd der Formel VIII hergestellt werden. Auch in diesem Fall handelt es sich um eine an sich bekannte und jedem Fachmann geläufige Reaktion. Sofern Ra Aryl oder Heteroaryl bedeutet, wird die Reaktion vorzugsweise in Gegenwart eines sekundären Amins, insbesondere eines cyclischen Amins, wie Piperidin oder Morpholin, durchgeführt. Als Lösungsmittel eignen sich beispielsweise niedere Alkohole, wie Methanol, Aethanol, 1-Propanol und 2-Propanol, aromatische Kohlenwasserstoffe, wie Benzol und Toluol, offenkettige und cyclische Aether, wie

Aethylenglykoldimethyläther, Tetrahydrofuran und Dioxan, halogenierte niedere Kohlenwasserstoffe, wie Methylenchlorid, N,N-Dimethylformamid und Dimethylsulfoxid. Man arbeitet vorzugsweise bei der Siedetemperatur des gewählten Lösungsmittels. In einer bevorzugten Ausführungsform verwendet man als Lösungsmittel einen Schlepper, beispielsweise einen aromatischen Kohlenwasserstoff oder einen halogenierten niederen Kohlenwasserstoff, und entfernt das gebildete Reaktionswasser mit einem Wasserabscheider.

Sofern Ra eine basische Aminogruppe bedeutet, wird der Aldehyd der Formel VIII vorzugsweise in Form eines entsprechenden Di(niederen Alkyl)acetals eingesetzt, wobei die vorerwähnten Lösungsmittel auch für den vorliegenden Fall geeignet sind. Vorzugsweise verwendet man einen niederen Alkohol, wie 1-Propanol, einen halogenierten niederen Kohlenwasserstoff, wie Methylenchlorid, oder Dimethylformamid als Lösungsmittel. Die Reaktionstemperatur ist nicht kritisch. Man kann in einem Bereich von etwa Raumtemperatur bis zur Siedetemperatur des gewählten Lösungsmittels arbeiten. Vorzugsweise arbeitet man jedoch in einem Bereich von etwa Raumtemperatur bis etwa 100°C.

Gemäss Verfahrensvariante d) können Verbindungen der Formel II, worin $R^3$ eine basische Aminogruppe bedeutet, durch Umsetzen einer Verbindung der Formel IIb mit Ammoniak oder einem primären oder sekundären basischen Amin hergestellt werden. Auch in diesem Fall handelt es sich um eine an sich bekannte und jedem Fachmann geläufige Reaktion. Geeignete Lösungsmittel sind beispielsweise halogenierte niedere Kohlenwasserstoffe, wie Methylenchlorid, offenkettige und cyclische Aether, wie Diäthyläther, t-Butylmethyläther, Tetrahydrofuran und Dioxan, N,N-Dimethylformamid, Acetonitril und Dimethylsulfoxid. Bevorzugte Lösungsmittel sind N,N-Dimethylformamid und Acetonitril. Die Reaktionstemperatur ist nicht kritisch, und man kann beispielsweise in einem Bereich von etwa 0°C bis zur Siedetemperatur des gewählten Lösungsmittels arbeiten. Vorzugsweise arbeitet man jedoch bei Raumtemperatur.

Die als Ausgangsstoffe verwendeten Verbindungen der Formel IVa, können beispielsweise gemäss dem nachfolgenden Reaktionsschema I hergestellt werden, worin $R^3$, $R^5$, $Y^-$ und die gestrichelte Linie obige Bedeutung besitzen:

Reaktionsschema I

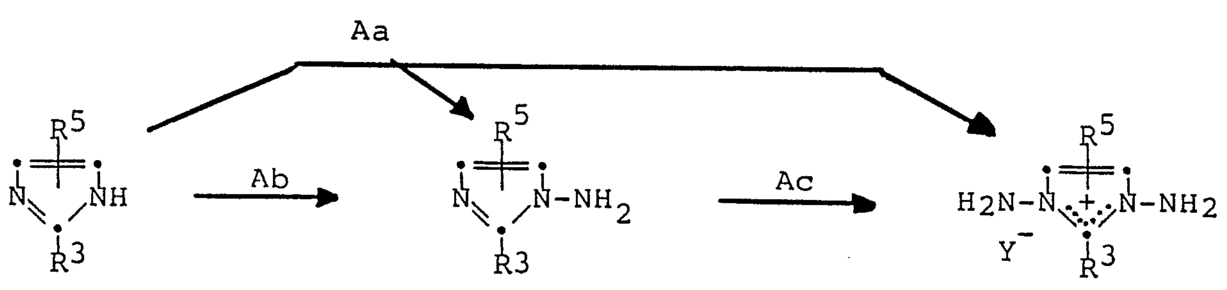


IXa Xa IVa

Verbindungen der Formel IVa, worin $R^3$ niederes Alkylthio bedeutet, können beispielsweise auch gemäss dem nachfolgenden Reaktionsschema II hergestellt werden, worin $R^5$, R", $Y^-$ und die gestrichelte Linie obige Bedeutung besitzen, und $\phi$ Phenyl bedeutet:

Reaktionsschema II

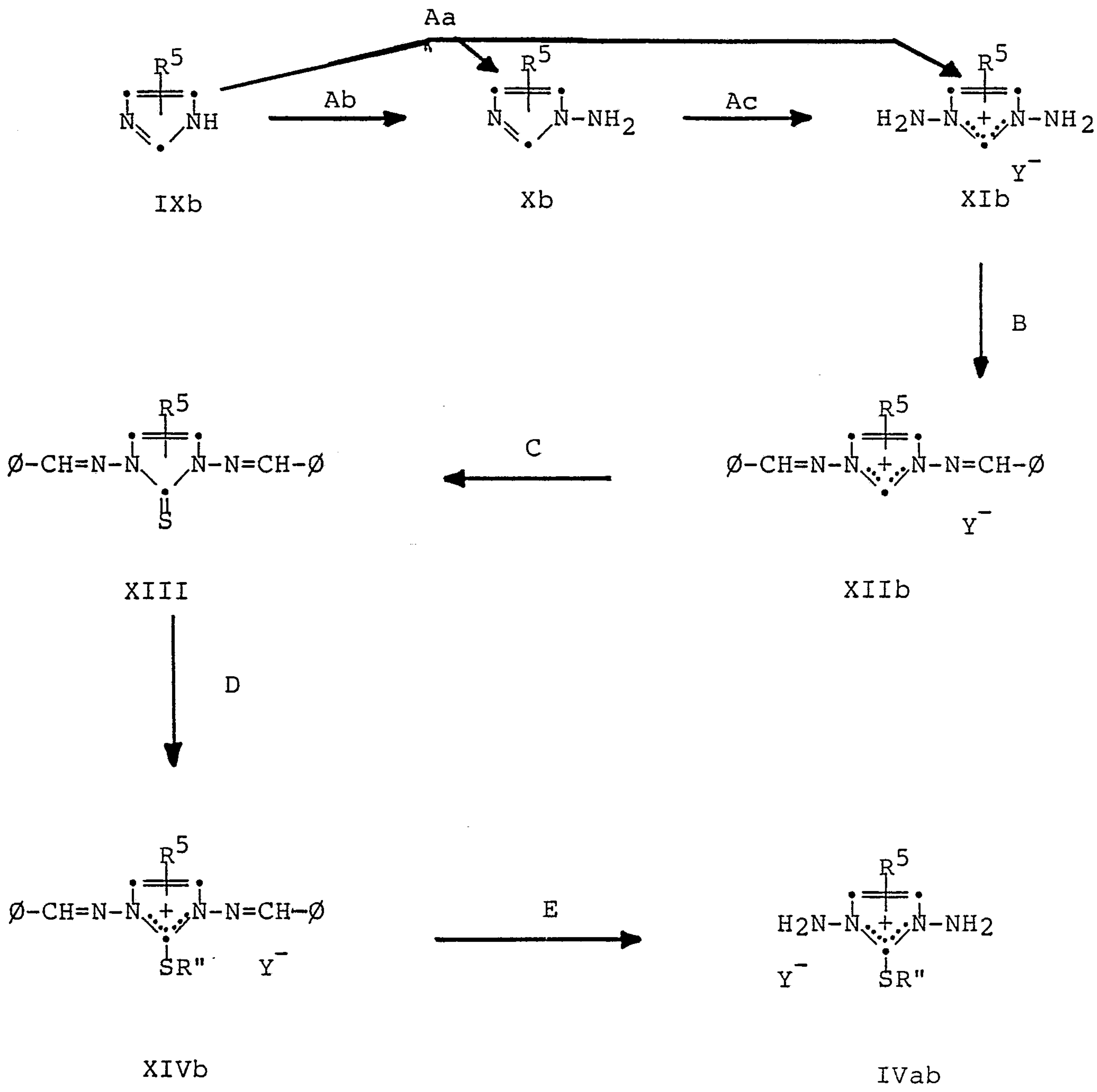


IXb Xb XIb XIII XIIb XIVb IVab

Verbindungen der Formel IVa, worin $R^3$ die Gruppe -CH=CH-Ra bedeutet, können beispielsweise auch gemäss dem nachfolgenden Reaktionsschema III hergestellt werden, worin $R^5$, Ra, $Y^-$, die gestrichelte Linie und $\phi$ obige Bedeutung besitzen:

## Reaktionsschema III

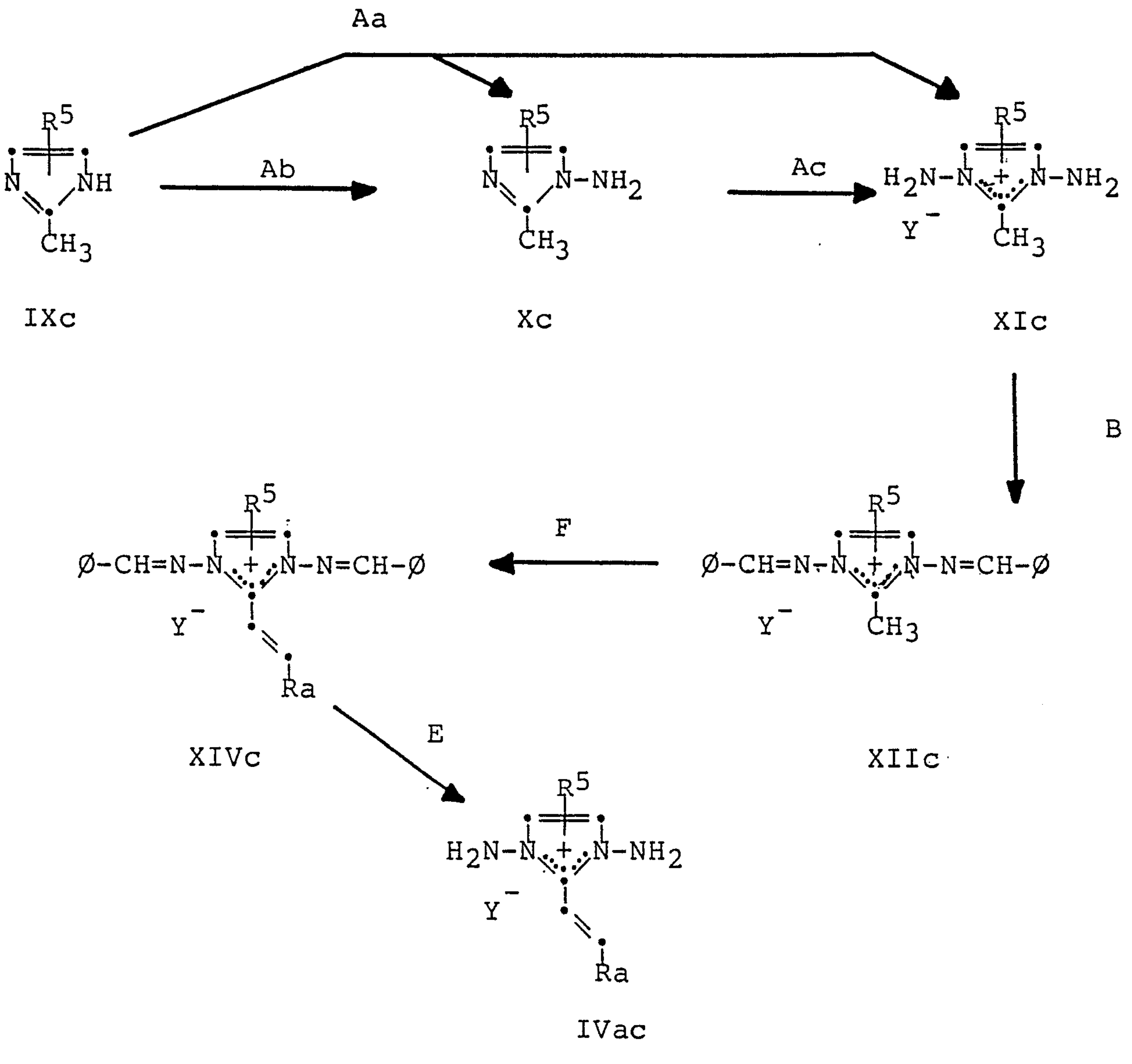


Verbindungen der Formel IVa, worin $R^3$ eine basische Aminogruppe bedeutet, können beispielsweise auch gemäss dem nachfolgenden Reaktionsschema IV hergestellt werden, worin $R^5$, $Y^-$, die gestrichelte Linie und $\phi$ obige Bedeutung besitzen, und Ra″ eine basische Aminogruppe bedeuten:

## Reaktionsschema IV

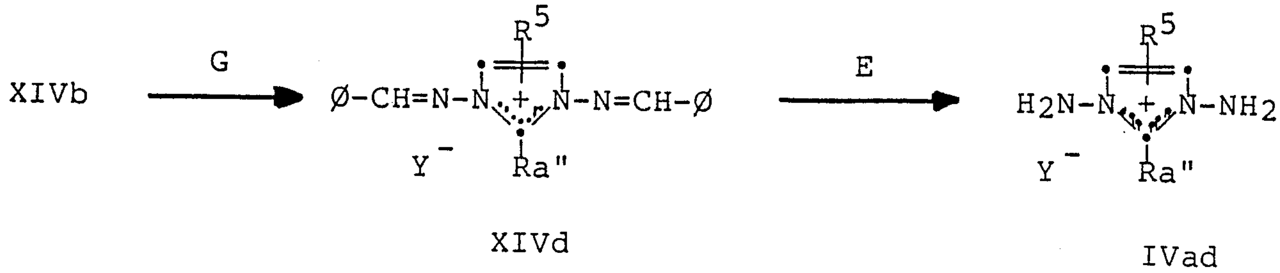

Die Verbindungen der Formel IVa, worin $R^3$ die Gruppe -A'-Ra", A' niederes Alkylen und Ra" eine basische Aminogruppe bedeuten, können beispielsweise auch gemäss dem nachfolgenden Reaktionsschema V hergestellt werden, worin $R^5$, Ra", A', $\phi$, $Y^-$ und die gestrichelte Linie obige Bedeutung besitzen, und X' ein Halogenatom bedeutet:

## Reaktionsschema V

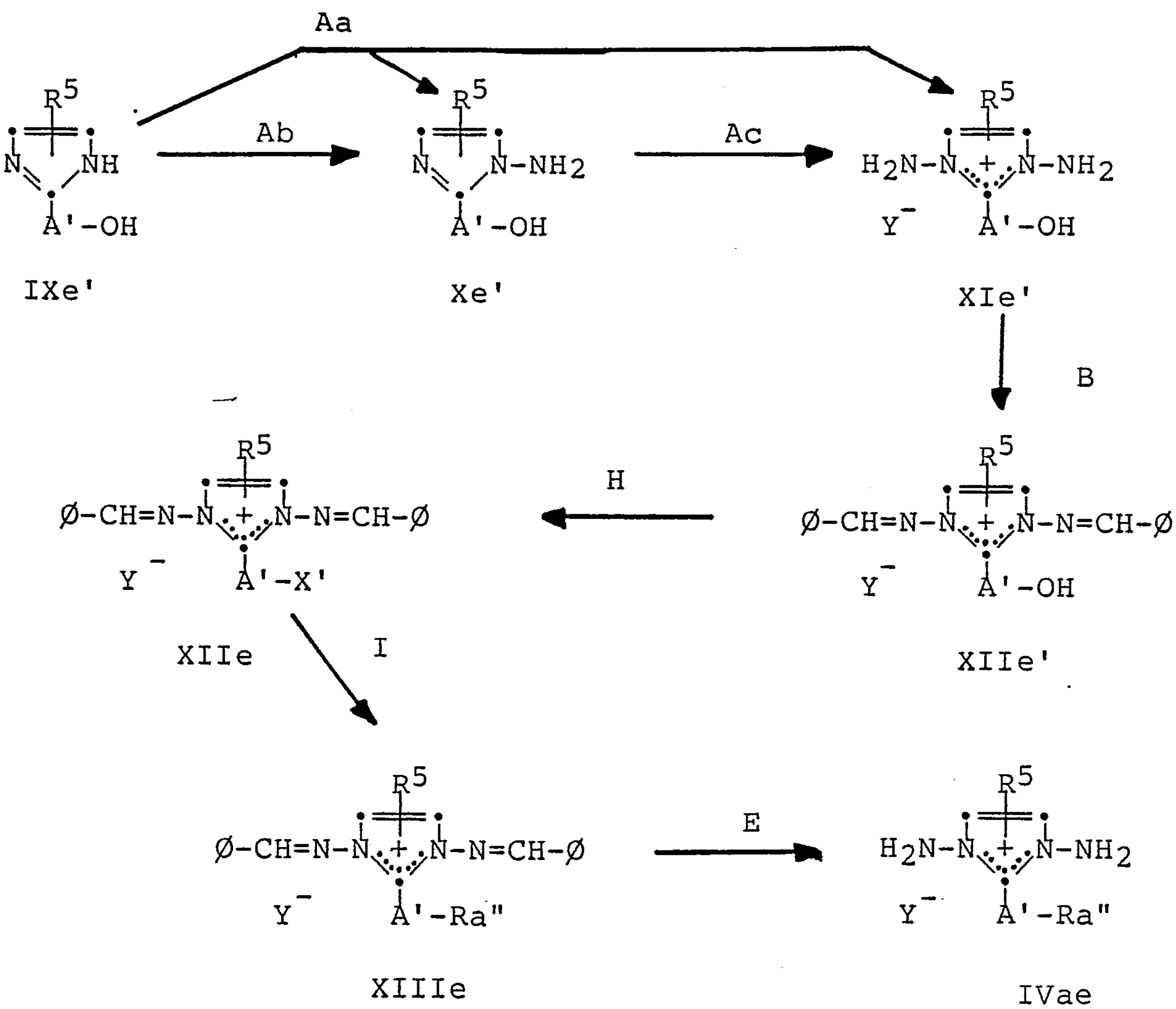


IXe' Xe' XIe' XIIe' XIIe XIIIe IVae

Die Verbindungen der Formel IVb können gemäss den vorstehenden Reaktionsschemata I-V hergestellt werden, indem man jeweils an Stelle einer Verbindung der Formel X eine entsprechende Verbindung der allgemeinen Formel

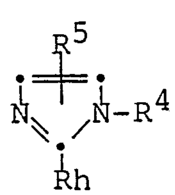

XV

worin Rh Wasserstoff, Methyl, die Gruppe -A'-OH oder $R^3$ bedeutet, und A', $R^3$, $R^4$ und $R^5$ obige Bedeutung besitzen,
als Ausgangsstoff einsetzt.

Die Verbindungen der Formel IVb, worin $R^4$ eine gesättigte oder partiell ungesättigte niedere Kohlenwasserstoffgruppe oder die Gruppe -CHRcRd oder -CHRc-CO-Re bedeutet, können beispielsweise auch gemäss dem nachfolgenden Reaktionsschema VI hergestellt werden, worin $R^3$, $R^{41}$, $R^5$, $Y^-$, $\phi$ und die gestrichelte Linie obige Bedeutung besitzen:

Reaktionsschema VI

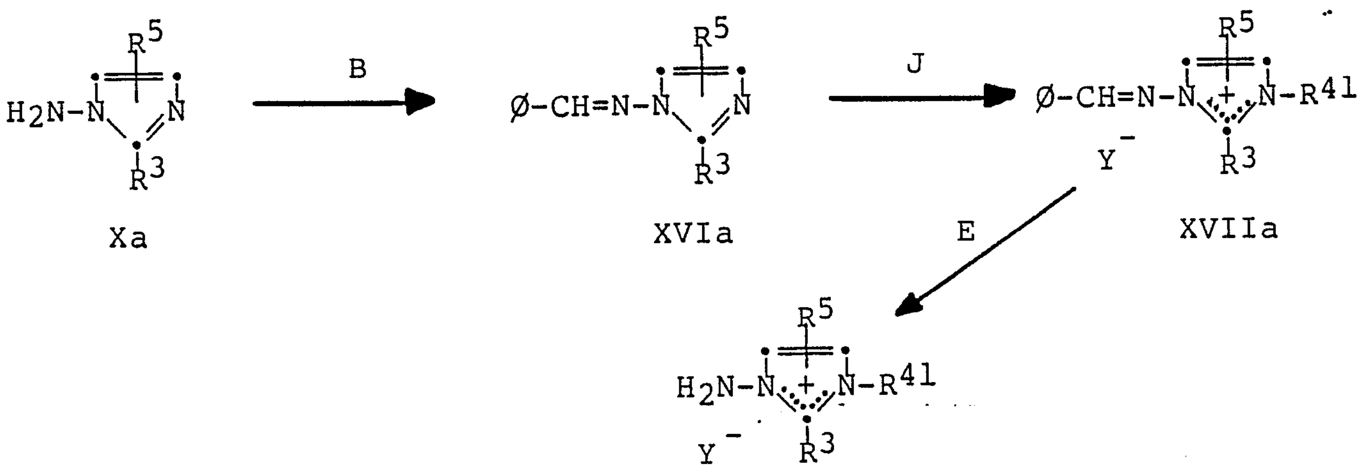


Xa XVIa XVIIa

IVba

Die Verbindungen der Formel IVb, worin $R^4$ eine gesättigte oder partiell ungesättigte niedere Kohlenwasserstoff gruppe oder die Gruppe -CHRcRd oder -CHRc-CO-Re und $R^3$ die Gruppe -SR″, -CH = CH-Ra, Ra″ oder -A′-Ra″ bedeuten, können auch hergestellt werden, indem man eine Verbindung der Formel Xb, Xc oder Xe′ gemäss vorstehendem Reaktionsschema VI an Stelle der Verbindung der Formel Xa in die der Formel XVIIa entsprechende Verbindung überführt und diese an Stelle der Verbindungen der Formel XII gemäss dem Reaktionsschema II, III, IV und V weiterverarbeitet.

Nachstehend werden die in den Reaktionsschemata erwähnten Reaktionsschritte A-G näher erläutert. Es handelt sich dabei durchwegs um an sich bekannt und jedem Fachmann geläufige Reaktionen.

Aa: Bei diesem Reaktionsschritt handelt es sich um eine elektrophile Aminierung mit einem Aminierungsmittel wie O-(2,4-Dinitrophenyl)hydroxylamin, O-Mesitylensulfonylhydroxylamin oder Hydroxylamin-O-sulfonsäure oder dessen Salzen mit anorganischen Basen. Vorzugsweise verwendet man die entsprechenden Alkalimetallsalze der Hydroxylamin-O-sulfonsäure, beispielsweise das Natriumsalz, und arbeitet in wässriger Lösung. Man erhält dabei in der Regel ein Gemisch bestehend aus dem Diamin der Formel IVa bzw. XI und dem Monoamin der Formel X. Die beiden Verbindungen können nach an sich bekannten und jedem Fachmann geläufigen Methoden voneinander getrennt werden. Die weiter unten folgenden Beispiele enthalten detaillierte Angaben betreffend die Auftrennung der erhaltenen Gemische.

Ab: Bei diesem Reaktionsschritt handelt es sich um eine elektrophile Aminierung mit einem Aminierungsmittel wie O-Diphenylphosphinylhydroxylamin oder O-Mesitylensulfonylhydroxylamin, wobei man das Ausgangsmaterial vor der Umsetzung mit dem Aminierungsmittel mit einer starken Base in ein Alkalimetallsalz überführt. Geeignete Basen sind beispielsweise niedere Alkalimetallalkoxide, wie Natriummethoxid und Natriumäthoxid, und Alkalimetallhydride, wie Natriumhydrid. Geeignete Lösungsmittel sind beispielsweise N-Methylpyrrolidon, N,N-Dimethylformamid, niedere Alkohole, Aether, wie Tetrahydrofuran, Diäthylenglykoldimethyläther, Diäthylenglykoldiäthyläther und Diäthylenglykoldibutyläther. Die Reaktionstemperatur liegt vorzugsweise in einem Bereich von etwa 0°C bis 100°C, wobei man vorzugsweise bei Raumtemperatur arbeitet.

Ac: Bei diesem Reaktionsschritt handelt es sich um eine elektrophile Aminierung mit einem Aminierungsmittel wie O-Diphenylphosphinylhydroxylamin oder O-Mesitylensulfonylhydroxylamin. Geeignete Lösungsmittel sind beispielsweise halogenierte niedere Kohlenwasserstoffe, wie Chloroform, Methylenchlorid und Aethylenchlorid, Aether, wie Diäthyläther und Tetrahydrofuran, niedere Alkohole, wie Aethanol,

Acetonitril, N,N-Dimethylformamid, Dimethylsulfoxid und Mischungen davon. Die Reaktionstemperatur liegt in einem Bereich von etwa 0°C bis zur Siedetemperatur des gewählten Lösungsmittels. Man arbeitet vorzugsweise bei Raumtemperatur.

B: Bei diesem Reaktionsschritt wird das entsprechende Ausgangsmaterial mit Benzaldehyd zum entsprechenden Aldimin umgesetzt. Geeignete Lösungsmittel sind beispielsweise niedere Fettsäuren, wie Essigsäure und Propionsäure, niedere Alkohole, wie Methanol, Aethanol und 2-Propanol, saure wässrige Medien, halogenierte niedere Kohlenwasserstoffe, wie Methylenchlorid, Chloroform und Aethylenchlorid, offenkettige und cyclische Aether, wie Diäthyläther, Diisopropyläther, t-Butylmethyläther, Aethylenglykoldimethyläther, Diäthylenglykoldiäthyläther, Tetrahydrofuran und Dioxan, Acetonitril, N,N-Dimethylformamid und Dimethylsulfoxid. Die Reaktion kann in einem Temperaturbereich von etwa 0°C bis zur Siedetemperatur des gewählten Lösungsmittels durchgeführt werden. Man arbeitet vorzugsweise bei Raumtemperatur.

C: Die gewünschte Thioxogruppe kann beispielsweise durch Behandeln des Ausgangsstoffes mit elementarem Schwefel in Gegenwart eines tertiären Amins und in einem geeigneten Lösungsmittel, beispielsweise in Pyridin, eingeführt werden. Die Reaktion kann in einem Bereich von etwa Raumtemperatur bis zur Siedetemperatur der Reaktionsmischung durchgeführt werden.

D: Die gewünschte Alkylierung kann beispielsweise durch Behandeln des Ausgangsstoffes mit einem Alkylierungsmittel wie einem Tri(niederen Alkyl)oxoniumtetrafluoroborat, z.B. mit Trimethyloxoniumtetrafluoroborat, bewerkstelligt werden. Als Lösungsmittel eignen sich beispielsweise halogenierte niedere Kohlenwasserstoffe, wie Methylenchlorid. Man arbeitet vorzugsweise bei Raumtemperatur.

E: Bei diesem Reaktionsschritt handelt es sich um die Hydrolyse eines Aldimins. In einer bevorzugten Ausführungsform behandelt man das entsprechende Ausgangsmaterial mit einer wässrigen Säure und entfernt den erhaltenen aromatischen Aldehyd mittels Wasserdampfdestillation. Geeignete Säuren sind beispielsweise verdünnte Salzsäure und verdünnte Bromwasserstoffsäure und Tetrafluorborsäure.

F: Bei dieser Reaktion wird eine Verbindung der Formel XIIc mit einem Aldehyd der Formel VIII kondensiert. Sofern Ra Aryl oder Heteroaryl bedeutet, wird die Reaktion vorzugsweise in Gegenwart eines sekundären Amins, insbesondere eines cyclischen Amins, wie Piperidin oder Morpholin, durchgeführt. Als Lösungsmittel eignen sich beispielsweise niedere Alkohole, wie Methanol, Aethanol, 1-Propanol und 2-Propanol, aromatische Kohlenwasserstoffe, wie Benzol und Toluol, offenkettige und cyclische Aether, wie Aethylenglykoldimethyläther, Tetrahydrofuran und Dioxan, halogenierte niedere Kohlenwasserstoffe, wie Methylenchlorid, N,N-Dimethylformamid und Dimethylsulfoxid. Man arbeitet vorzugsweise bei der Siedetemperatur des gewählten Lösungsmittels. In einer bevorzugten Ausführungsform verwendet man als Lösungsmittel einen Schlepper, beispielsweise einen aromatischen Kohlenwasserstoff oder einen halogenierten niederen Kohlenwasserstoff, und entfernt das gebildete Reaktionswasser mit einem Wasserabscheider.

Sofern Ra eine basische Aminogruppe bedeutet, wird der Aldehyd der Formel VIII vorzugsweise in Form eines entsprechenden Di(niederen Alkyl)acetals eingesetzt, wobei die vorerwähnten Lösungsmittel auch für den vorliegenden Fall geeignet sind. Vorzugsweise verwendet man einen niederen Alkohol, wie 1-Propanol, einen halogenierten niederen Kohlenwasserstoff, wie Methylenchlorid, oder Dimethylformamid als Lösungsmittel. Die Reationstemperatur ist nicht kritisch. Man kann in einem Bereich von etwa Raumtemperatur bis zur Siedetemperatur des gewählten Lösungsmittels arbeiten. Vorzugsweise arbeitet man jedoch in einem Bereich von etwa Raumtemperatur bis etwa 100°C.

G: Bei dieser Reaktion wird eine Verbindung der Formel XIVb mit Ammoniak oder einem primären oder sekundären basischen Amin umgesetzt. Geeignete Lösungsmittel sind beispeilsweise halogenierte niedere Kohlenwasserstoffe, wie Methylenchlorid, offenkettige und cyclische Aether, wie Diäthyläther, t-Butylmethyläther, Tetrahydrofuran und Dioxan, N,N-Dimethylformamid, Acetonitril und Dimethylsulfoxid. Bevorzugte Lösungsmittel sind N,N-Dimethylformamid und Acetonitril. Die Reaktionstemperatur ist nicht kritisch, und man kann beispielsweise in einem Bereich von etwa 0°C bis zur Siedetemperatur des gewählten Lösungsmittels arbeiten. Vorzugsweise arbeitet man jedoch bei Raumtemperatur.

H: Bei dieser Reaktion wird eine Verbindung der Formel XIIe' mit einem Halogenierungsmittel wie Thionylchlorid und Phosphoroxychlorid umgesetzt, wobei man als Lösungsmittel vorzugsweise überschüssiges Halogenierungsmittel einsetzt. Die Reaktion kann in einem Temperaturbereich von etwa 0°C bis zur Siedetemperatur der Reaktionsmischung durchgeführt werden.

I: Bei dieser Reaktion wird eine Verbindung der Formel XIIe mit Ammoniak oder einem primären oder sekundären basischen Amin umgesetzt, wobei man als Lösungsmittel vorzugsweise Aceton oder N,N-Dimethylformamid verwendet. Die Reaktion kann in einem Temperaturbereich von etwa Raumtemperatur bis zur Siedetemperatur der Reaktionsmischung durchgeführt werden.

J: Bei diesem Reaktionsschritt wird das entsprechende Ausgangsmaterial mit einer Verbindung der obigen Formel VI umgesetzt. Geeignete Lösungsmittel sind beispielsweise halogenierte niedere Kohlenwasserstoffe, wie Methylenchlorid, Chloroform und Aethylenchlorid, offenkettige und cyclische Aether, wie Diäthyläther, Diisopropyläther, Aethylenglykoldimethyläther, Diäthylenglykoldiäthyläther, Tetrahydrofuran und Dioxan, Acetonitril und N,N-Dimethylformamid. Diese Reaktion kann in einem Temperaturbereich von etwa 0°C bis zur Siedetemperatur des gewählten Lösungsmittels durchgeführt werden.

Die als Ausgangsstoffe verwendeten Verbindungen der Formel VI können in Analogie zur Verfahrensvariante a) oder zum weiter oben beschriebenen Reaktionsschritt B durch Umsetzen einer Verbindung der Formel Xa mit einem Aldehyd oder Keton der Formel V hergestellt werden.

Wie bereits eingangs erwähnt besitzen die den Imidazoliumhydrogencarbonaten der Formel I entsprechenden pharmazeutisch annehmbaren Salze, d.h. die Verbindungen der Formel III, wertvolle pharmakologische Eigenschaften. Sie weisen beispielsweise antiparasitäre Eigenschaften auf und sind insbesondere wirksam gegen parasitäre Protozoen und Würmer. Besonders ausgeprägt ist ihre Wirkung gegen parasitäre Würmer, insbesondere gegen Nematoden wie die Filarien. Diese pharmakologischen Eigenschaften können mittels bekannter und jedem Fachmann geläufiger Testmethoden ermittelt werden.

Die filarizide Wirkung der Verbindungen der Formel III kann beispielsweise an Baumwollratten (Sigmodon hispidus) ermittelt werden, welche mit Litomosoides carinii infiziert worden sind. Die Infektion mit L. carinii wird durch die blutsaugende Milbe Bdellonyssus bacoti übertragen, in welcher sich die Entwicklung von der Mikrofilarie zur infektiösen Larve vollzieht. Die Baumwollratten werden infiziert, indem man sie dem Biss infizierter Milben aussetzt. 14 Wochen nach der Infektion werden Gruppen von 2-4 Tieren subkutan mit der zu testenden Verbindung behandelt. 42 Tage nach Behandlung mit der zu testenden Verbindung werden die Versuchstiere seziert, wobei man die adulten Filarien aus der Pleuralhöhle entfernt. Lebende und tote oder eingekapselte Würmer werden voneinander getrennt und gewogen. Die filarizide Wirkung wird ausgedrückt als prozentualer Anteil toter Makrofilarien pro Behandlungsgruppe. Mit den Werten aus verschiedenen Dosierungsgruppen wird dann die $ED_{90}$ mittels Probitanalyse bestimmt. Die $ED_{90}$ ist diejenige Dosis, bei welcher 90% der aus der Pleuralhöhle entfernten Würmer tot sind. In der nachfolgenden Tabelle werden die Resultate zusammengestellt, welche mit repräsentativen Vertretern der durch die allgemeine Formel III definierten Verbindungsklasse in dem vorstehend geschilderten Versuch erhalten worden sind. Die Tabelle enthält ausserdem Angaben über die akute Toxizität dieser Verbindungen in mg pro kg bei einmaliger oraler Verabreichung an Mäusen.

## Tabelle

| Verbindung | $ED_{90}$ in mg/kg s.c. | $DL_{50}$ in mg/kg p.o. |
|---|---|---|
| A | 1,0 | 312- 625 |
| B | 0,5 | - |
| C | 0,85 | - |
| D | <1,0 | 1250-2500 |

Verbindung A = 1,3-Bis[[p-(Dimethylamino)benzyliden]-amino]-2-[2-(dimethylamino)vinyl]-imidazoliumchlorid.

Verbindung B = 1,3-Bis[[p-(Dimethylamino)benzyliden]-amino]-2-isopropylimidazoliumchlorid.

Verbindung C = 1,3-Bis[[p-(Dimethylamino)benzyliden]-amino]-2-methylimidazoliumacetat.

Verbindung D = 1,3-Bis[[p-(Dimethylamino)benzyliden]-amino]-2-phenylimidazoliumchlorid.

Die Verbindungen der Formel III können als Heilmittel, z.B. in Form pharmazeutischer Präparate zur enteralen oder parenteralen Applikation, Verwendung finden. Sie können beispielsweise peroral, z.B. in Form von Tabletten, Lacktabletten, Dragées, Hart-und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die beschriebenen Verbindungen der Formel III, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragées und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Stabilisierungs-, Konservierungs-, Netz-und Emulgiermittel, Mittel zur geschmacklichen Verbesserung, Salze zur Veränderung des osmotischen Druckes, Puffersubstanzen, Lösungsvermittler, Färbe-und Ueberzugsmittel und Antioxidantien in Frage.

Die Dosierung der Verbindungen der Formel III kann, abhängig von der zu behandelnden Krankheit, dem Alter und dem individuellen Zustand des Patienten und von der Applikationsweise, innerhalb weiter Grenzen variieren und ist natürlich in jedem einzelnen Fall den individuellen Gegebenheiten anzupassen. Für die Prophylaxe und Therapie von Infektionskrankheiten, welche durch Bakterien, Pilze oder Parasiten hergerufen werden, kommt für den erwachsenen Patienten eine tägliche Dosis von etwa 0,01 g bis etwa 4 g, insbesondere etwa 0,05 g bis etwa 2 g in Betracht. Je nach Dosierung ist es dabei zweckmässig, die Tagesdosis in mehreren Dosierungseinheiten zu verabreichen.

Die pharmazeutischen Präparate enthalten zweckmässigerweise etwa 10-1000 mg, vorzugsweise 50-500 mg einer Verbindung der Formel III.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

i) 0,22 g (1,5 mMol) 1,3-Diamino-2-methylimidazoliumchlorid werden in 5 ml Eisessig gelöst, worauf man mit einer Lösung von 0,45 g (3 mMol) 4-Dimethylaminobenzaldehyd in 5 ml Eisessig versetzt. Nach Rühren während 2 Stunden wird der gelbe Niederschlag abfiltriert. Nach dem Trocknen erhält man 1,3-Bis[-[p-(dimethylamino)benzyliden]amino] -2-methylimidazoliumchlorid von Zersetzungspunkt 264-266°. Die Mutterlauge wird mit Aether versetzt, wobei eine zweite Portion des gewünschten Produktes erhalten wird. Das erhaltene Material wird aus heissem Wasser umkristallisiert. Man erhält 1,3-Bis[[p-(dimethylamino)-benzyliden]amino] -2-methylimidazoliumchlorid vom Zersetzungspunkt 270°.

ii) 2,02 g 1,3-Diamino-imidazoliumchlorid und 4,5 g 4-Dimethylaminobenzaldehyd werden bei 50° in 100 ml Eisessig gelöst, worauf man während 15 Stunden bei Raumtemperatur rührt. Es entstehen gelbe Kristalle. Man gibt 100 ml Aether dazu, nutscht unter Waschen mit 100 ml Aether ab und trocknet das erhaltene Material bei 60°. Durch Umkristallisieren aus 250 ml Aethanol erhält man 1,3-Bis[[(p-dimethylamino) benzyliden]amino] imidazoliumchlorid vom Schmelzpunkt 244°.

a) Man suspendiert 3,28 g (8 mMol) 1,3-Bis[[p-(dimethylamino)benzyliden]amino] -2-methylimidazoliumchlorid in 200 ml Chloroform und 400 ml gesättigter Natriumhydrogencarbonatlösung und rührt 18 Stunden bei Raumtemperatur. Das Produkt wird abfiltriert und mit Wasser gewaschen. Man erhält 1,3-Bis[-[p-(dimethylamino)benzyliden]amino] -2-methylimidazoliumhydrogencarbonat vom Schmelzpunkt 144-146° (Zers.).

In analoger Weise erhält man:

b) Aus 1,3-Bis[[p-(dimethylamino)benzyliden]amino]imidazoliumchlorid das 1,3-Bis[[p-(dimethylamino)-benzyliden]amino] imidazoliumhydrogencarbonat vom Schmelzpunkt 103-104° (Zers.).

## Beispiel 2

i) Zu einer Lösung von 0,88 g (5 mMol) 1,3-Diamino-2-isopropyl-imidazoliumchlorid in 15 ml Eisessig werden 1,49 g (10 mMol) 4-Dimethylamino-benzaldehyd gegeben. Nach Stehenlassen bei Raumtemperatur während 2 Tagen wird das Produkt durch Zugabe von Aether auskristallisiert und dann aus Aethanol umkristallisiert. Man erhält 1,3-Bis[[p-(dimethylamino)benzyliden]amino]-2-isopropyl-imidazoliumchlorid vom Schmelzpunkt 241°.

ii.a) Zu einer Lösung von 1,99 g (10 mMol) 1-Benzylidenamino-2-äthylimidazol in 10 ml Acetonitril gibt man 2,16 g (10 mMol) 3,4,5-Trimethoxybenzylchlorid und erhitzt während 28 Stunden unter Rückfluss. Das auskristallisierte Produkt wird abfiltriert und mit Aether gewaschen. Man erhält 1-Benzylidenamino-2-äthyl-3-(3,4,5 -trimethoxybenzyl)imidazoliumchlorid vom Schmelzpunkt 205°.

ii.b) 3,1 g (7,45 mMol) 1-Benzylidenamino-2-äthyl-3-(3,4,5 -trimethoxybenzyl)imidazoliumchlorid werden in 70 ml Wasser gelöst, worauf man mit 8 ml 25-proz. Salzsäure versetzt. Der entstehende Benzaldehyd wird mittels Wasserdampfdestillation entfernt. Nach dem Eindampfen wird das Produkt aus Aethanol/Aether umkristallisiert. Man erhält 1-Amino-2-äthyl-3-(3,4,5 -trimethoxybenzyl)imidazoliumchlorid vom Schmelzpunkt 174°.

ii.c) 1,9 g (5,8 mMol) 1-Amino-2-äthyl-3-(3,4,5 -trimethoxybenzyl)imidazoliumchlorid werden in 38 ml Eisessig gelöst, worauf man mit 0,86 g (5,8 mMol) p-Dimethylaminobenzaldehyd versetzt, und während 52 Stunden bei Raumtemperatur rührt und die Lösung eindampft. Das Produkt wird aus Aethanol/Aether umkristallisiert. Man erhält 2-Aethyl-1-[[p-(dimethylamino)benzyliden]amino]-3-(3,4,5-trimethoxybenzyl)-imidazoliumchlorid vom Schmelzpunkt 211°.

iii) Eine Lösung von 2,42 g (10 mMol) 1-[[p-(Dimethylamino)-benzyliden]amino]-2-äthylimidazol wird mit 2,42 g (10 mMol) p-Dimethylaminophenacylbromid versetzt. Nach Rühren während 30 Minuten bei 40° wird das Produkt durch Zugabe von Aether auskristallisiert und aus Methylenchlorid/Aether umkristallisiert. Man erhält 3-[p-(Dimethylamino)phenacyl] -1-[[p-(dimethylamino)benzyliden]amino]-2-äthylimidazoliumbromid vom Schmelzpunkt 247°.

a) man suspendiert 3,0 g (6,8 mMol) 1,3-Bis[[p-(dimethylamino)benzyliden]amino] -2-isopropylimidazoliumchlorid in 200 ml gesättigter Natriumhydrogencarbonatlösung und rührt 18 Stunden bei Raumtemperatur. Das Produkt wird abfiltriert und zuerst mit Wasser und dann mit Aether gewaschen. Man erhält 1,3-Bis[-[p-(dimethylamino)benzyliden]amino] -2-isopropylimidazoliumhydrogencarbonat vom Schmelzpunkt 124-126° (Zers.).

In analoger Weise erhält man:

b) Aus 2-Aethyl-1-[[p-(dimethylamino)benzyliden] -3-(3,4,5-trimethoxybenzyl)imidazoliumchlorid das 2-Aethyl-1-[[p-(dimethylamino)benzyliden] -3-(3,4,5-trimethoxybenzyl)imidazoliumhydrogencarbonat vom Schmelzpunkt 75° (Zers.);

c) aus 3-[p-(Dimethylamino)phenacyl]-3-[[p-(dimethylamino) benzyliden]amino]-2-äthylimidazoliumbromid das 3-[p-(Dimethylamino)phenacyl] -3-[[p-(dimethylamino)benzyliden]amino] -2-äthylimidazoliumhydrogencarbonat vom Schmelzpunkt 122° (Zers.).

## Beispiel 3

a) Man löst 1,3 g (2,6 mMol) 1,3-Bis[[p-(dimethylamino)benzyliden]amino] -2-isopropylimidazoliumhydrogencarbonat in 10 ml Methanol und gibt 2 ml Eisessig dazu. Nach 16 Stunden Rühren bei Raumtemperatur wird die Lösung konzentriert und das Produkt durch Zugabe von Aether kristallisiert. Nach zweimaligem Umkristallisieren aus Methanol/Aether erhält man 1,3-Bis[[p-(dimethylamino)benzyliden]amino] -2-isopropylimidazoliumacetat vom Schmelzpunkt 150-151°.

In analoger Weise erhält man:

b) Aus 1,3-Bis[[p-(dimethylamino)benzyliden]amino]-2-methylimidazoliumhydrogencarbonat das 1,3-Bis[[p-(dimethylamino)benzyliden]amino] -2-methylimidazoliumacetat vom Schmelzpunkt 159-162°.

Beispiel 4

a) Man löst 48 mg (0,1 mMol) 1,3-Bis[[p-(dimethylamino)-benzyliden]amino] -2-isopropylimidazoliumhydrogencarbonat in 4 ml Methanol und gibt 9,6 mg (0,1 mMol) Methansulfonsäure dazu. Nach 10 Minuten konzentriert man die Lösung bei Raumtemperatur im Vakuum und kristallisiert das Produkt durch Zugabe von Aether. Man erhält 1,3-Bis[[p-(dimethylamino)-benzyliden]amino] -2-isopropylimidazoliummethansulfonat vom Schmelzpunkt 238-239°.

In analoger Weise erhält man:

b) Aus 1,3-Bis[[p-(dimethylamino)benzyliden]amino]-2-methylimidazoliumhydrogencarbonat das 1,3-Bis[[p-(dimethylamino)benzyliden]amino] -2-methylimidazoliummethansulfonat vom Schmelzpunkt 255-257° (Zers.).

Beispiel 5

Man suspendiert 25 mg (0,05 mMol) 1,3-Bis[[p-(dimethylamino)benzyliden]amino] -2-isopropylimidazoliumacetat in 5 ml gesättigter Natriumhydrogencarbonatlösung. Nach drei Tagen Rühren wird das Produkt abfiltriert und mit Wasser gewaschen. Man erhält 1,3-Bis[[p-(dimethylamino)benzyliden]-amino] -2-isopropylimidazoliumhydrogencarbonat vom Schmelzpunkt 122-124°.

Beispiel 6

Man suspendiert 100 mg (0,2 mMol) 1,3-Bis[[p-(dimethylamino)benzyliden]amino] -2-isopropylimidazoliumhydrogencarbonat in 3 ml Wasser und gibt 2 ml 10-proz. Bromwasserstofflösung dazu. Nach 15 Minuten wird das Produkt abfiltriert, mit Wasser gewaschen und aus Aethanol umkristallisiert. Man erhält 1,3-Bis[[p-(dimethylamino)benzyliden]amino] -2-isopropylimidazoliumbromid vom Schmelzpunkt 241° (Zers.).

Beispiel 7

i) 2,05 g (5 mMol) 1,3-Bis[[p-(dimethylamino)benzyliden]-amino] -2-methylimidazoliumchlorid und 7,35 g (50 mMol) N,N-Dimethylformamiddiäthylacetal werden in 150 ml 1-Propanol während 45 Minuten bei 100° gerührt. Durch Zugabe von Aether wird das Produkt ausgefällt. Es wird aus Dichlormethan/Methanol kristallisiert und aus Aethanol umkristallisiert. Man erhält 1,3-Bis[[p-(dimethylamino)benzyliden]amino] -2-[2-(dimethylamino)vinyl]imidazoliumchlorid vom Schmelzpunkt 246-248°.

a) Man suspeniert 2,75 g (5,7 mMol) 1,3-Bis[[p-(dimethylamino)benzyliden]amino] -2-[2-(dimethylamino)vinyl]-imidazoliumchlorid in 100 ml gesättigter Natriumhydrogencarbonatlösung und rührt 16 Stunden bei Raumtemperatur. Das Produkt wird abfiltriert und mit Wasser gewaschen. Man erhält 1,3-Bis[-[p-(dimethylamino)benzyliden]amino]-2-[2-(dimethylamino)vinyl]imidazoliumhydrogencarbonat vom Schmelzpunkt 160-161°.

Beispiel 8

Man löst 1,0 g (1,9 mMol) 1,3-Bis[[p-(dimethylamino)benzyliden]amino] -2-[2-dimethylamino)vinyl]-imidazoliumhydrogencarbonat in 20 ml Methanol und 3 ml Eisessig. Nach 20 Minuten wird die Lösung bei Raumtemperatur im Vakuum konzentriert. Das Produkt wird durch Zugabe von Aether kristallisiert und dreimal aus Methanol/Aether umkristallisiert. Man erhält 1,3-Bis[[p-(dimethylamino)benzyliden]amino] -2-[2-dimethylamino)vinyl]imidazoliumacetat vom Schmelzpunkt 182° (Zers.).

Beispiel 9

i.a) 32,8 g (0,75 Mol) Natriumhydrid (55-proz. Dispersion in Oel) werden mit absolutem Tetrahydrofuran gewaschen. Eine Lösung von 53,1 g (0,37 Mol) 2-Phenylimidazol in 1500 ml N-Methylpyrrolidon wird dann bei 0° dazugetropft. Man rührt eine halbe Stunde bei 0° und etwa während 1,5 Stunden bei

Raumtemperatur (bis keine Gasentwicklung mehr zu beobachten ist). 85 g (0,36 Mol) O-Diphenylphosphinylhydroxylamin werden nun dazugegeben. Man rührt das Gemisch während 20 Stunden bei Raumtemperatur und gibt anschliessend 910 ml Wasser dazu. Die dunkle Lösung wird 1 Stunde bei Raumtemperatur gerührt und dann siebenmal mit je 300 ml Dichlormethan extrahiert. Der Auszug wird über Natriumsulfat getrocknet und eingedampft. Das verbleibende Oel wird auf eine mit 900 g Kieselgel beladene Säule gebracht, worauf man mit Dichlormethan/Aethanol (85:15) das 1-Amino-2-phenylimidazol eluiert.

i.b) Zu 30,1 g (0,188 Mol) 1-Amino-2-phenylimidazol in 2000 ml Dichlormethan werden 43,8 g (0,188 Mol) O-Diphenylphosphinylhydroxylamin gegeben. Nach Rühren während 3 Tagen bei Raumtemperatur werden nochmals 21,9 g (0,094 Mol) O-Diphenylphosphinylhydroxylamin zugefügt. Nach einem Tag wird das Gemisch filtriert, und das Filtergut wird viermal mit je 150 ml Dichlormethan gewaschen. Filtrat und Waschlösung werden eingedampft. Der Rückstand wird auf eine mit 700 ml Ionenaustauscher Amberlite IRA 400 (Chlorid) beladene Säule gebracht, worauf man mit Wasser eluiert. Das Eluat wird eingedampft, und der Rückstand wird zweimal mit Aethanol versetzt und eingedampft. Nach Trocknen im Hochvakuum über Kaliumhydroxid erhält man 1,3-Diamino-2-phenylimidazoliumchlorid.

i.c) 0,45 g (2,1 mMol) 1,3-Diamino-2-phenylimidazoliumchlorid werden in 6 ml Eisessig gelöst. 0,64 g (4,3 mMol) 4-Dimethylaminobenzaldehyd werden dazugegeben. Nach Rühren während 24 Stunden wird etwa 1 ml absoluter Aether dazugegeben. Ein Tag später wird eingedampft, und der Rückstand wird auf eine mit 25 g Kieselgel beladene Säule gebracht. Nach Eluieren mit Dichlormethan/Aethanol (98:2) und Kristallisieren aus Aethanol/Aether erhält man 1,3-Bis[[p-(dimethylamino)benzyliden]amino] -2-phenylimidazoliumchlorid vom Schmelzpunkt 242°.

a) Man suspendiert 95 mg (0,2 mMol) 1,3-Bis[[p-(dimethylamino)benzyliden]amino] -2-phenylimidazoliumchlorid in 10 ml gesättigter Natriumhydrogencarbonatlösung und rührt 18 Stunden bei Raumtemperatur. Das Produkt (1,3-Bis[[p-(dimethylamino)benzyliden]amino] -2-phenylimidazoliumhydrogencarbonat) wird abfiltriert, mit Wasser und Aether gewaschen. Das noch feuchte Produkt wird anschliessend in 10 ml Methanol gelöst und mit 2 ml Eisessig versetzt. Die Lösung wird im Vakuum bei Raumtemperatur eingedampft und der Rückstand aus Methanol/Aether kristallisiert. Man erhält 1,3-Bis[[p-(dimethylamino)benzyliden]amino] -2-phenylimidazoliumacetat vom Schmelzpunkt 167°.

Beispiel A

1,3-Bis[[(p-dimethylamino)benzyliden]amino] -2-[2-(dimethylamino)vinyl]imidazoliumacetat wird in an sich bekannter Weise als Wirkstoff zur Herstellung von Tabletten nachfolgender Zusammensetzung verwendet:

| | | mg/Tablette |
|---|---|---|
| Wirkstoff | | 100 |
| Lactose | | 192 |
| Maisstärke | | 80 |
| Hydrolysierte Maisstärke | | 20 |
| Calciumstearat | | 8 |
| | Tablettengewicht | 400 mg |

Als Wirkstoffe können auch die nachfolgend aufgeführten Verbindungen verwendet werden:
1,3-Bis[[(p-dimethylamino)benzyliden]amino]-2-phenylimidazoliumacetat;
1,3-Bis[[p-(dimethylamino)benzyliden]amino]-2-isopropylimidazoliumacetat;
1,3-Bis[[p-(dimethylamino)benzyliden]amino]-2-isopropylimidazoliummethansulfonat;
1,3-Bis[[p-(dimethylamino)benzyliden]amino]-2-methylimidazoliumacetat und
1,3-Bis[[p-(dimethylamino)benzyliden]amino]-2-methylimidazoliummethansulfonat.

## Ansprüche

1. Verbindungen der allgemeinen Formel

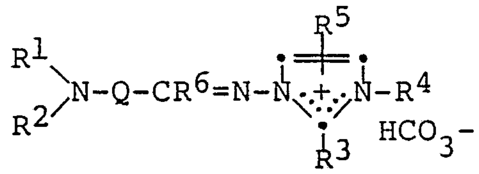

I

worin das Symbol Q Arylen oder Heteroarylen, die Gruppe $-NR^1R^2$ eine basische Aminogruppe, $R^3$ Wasserstoff, niederes Alkyl, niederes Hydroxylalkyl, niederes Alkoxyalkyl, niederes Halogenalkyl, niederes Alkylthio, niederes Alkoxy oder die Gruppe $-(A)_n$-Ra, $R^4$ eine gesättigte oder partiell ungesättigte niedere Kohlenwasserstoffgruppe, eine basische Aminogruppe oder die Gruppe -N = CRc-Rb, -CHRcRd, -NH-CHRcRd, -NH-CO-Re oder -CHRc-CO-Re, $R^5$ Wasserstoff, niederes Alkyl, niederes Hydroxylalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl-oder basische Aminogruppe, A Vinylen oder niederes Alkylen, n die Zahl 0 oder 1 und die gestrichelte Linie eine zusätzliche Doppelbindung bedeuten..

2. Verbindungen gemäss Anspruch 1, worin $R^3$ niederes Alkyl bedeutet.

3. Verbindungen gemäss Anspruch 1, worin $R^3$ die Gruppe $-(A)_n$-Ra bedeutet.

4. Verbindungen gemäss Anspruch 3, worin n die Zahl 0 und Ra eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeuten.

5. Verbindungen gemäss Anspruch 4, worin Ra eine unsubstituierte oder durch niederes Alkyl, niederes Alkoxy oder Halogen mono-substituierte Phenylgruppe bedeutet.

6. Verbindungen gemäss Anspruch 3, worin n die Zahl 1, A Vinylen, Ra die Gruppe -NRR′ und R und R′ je Wasserstoff oder niederes Alkyl oder zusammen mit dem Stickstoffatom einen gegebenenfalls durch eine oder zwei niedere Alkylgruppen substituierten, 5-oder 6-gliedrigen, gesättigten Heterocyclus, der ein oder zwei Stickstoffatome oder ein Stickstoffatom und ein Sauerstoff-oder Schwefelatom als Heteroatom enthält, bedeuten.

7. Verbindungen gemäss Anspruch 6, worin R und R′ je niederes Alkyl oder zusammen mit dem Stickstoffatom 4-Morpholinyl oder 1-Piperidinyl bedeuten.

8. Verbindungen gemäss einem der Ansprüche 1-7, worin $R^5$ Wasserstoff bedeutet.

9. Verbindungen gemäss einem der Ansprüche 1-8, worin $R^4$ die Gruppe $-N=CR^6-Q-NR^1R^2$ bedeutet.

10. Verbindungen gemäss einem der Ansprüche 1-8, worin $R^4$ die Gruppe -N = CRc-Rb bedeutet.

11. Verbindungen gemäss Anspruch 10, worin Rc Wasserstoff bedeutet.

12. Verbindungen gemäss Anspruch 10 oder 11, worin Rb eine unsubstituierte oder durch einen, zwei oder drei Substituenten aus der Gruppe bestehend aus niederem Alkyl, niederem Alkoxy, niederem Alkylthio, Halogen und Nitro substituierte Phenylgruppe bedeutet.

13. Verbindungen gemäss einem der Ansprüche 1-12, worin Q 1,4-Phenylen bedeutet, das noch durch einen oder zwei Substituenten aus der Gruppe bestehend aus niederem Alkyl und niederem Alkoxy substituiert sein kann.

14. Verbindungen gemäss Anspruch 13, worin Q 1,4-Phenylen bedeutet.

15. Verbindungen gemäss einem der Ansprüche 1-14, worin $R^1$ und $R^2$ je niederes Alkyl bedeuten.

16. Verbindungen gemäss einem der Ansprüche 1 bis 15, worin $R^6$ Wasserstoff bedeutet.

17. 1,3-Bis[[p-(dimethylamino)benzyliden]amino]-2-[2-(dimethylamino)vinyl]-imidazoliumhydrogencarbonat.

18. 1,3-Bis[[p-(dimethylamino)benzyliden]amino]-2-isopropylimidazoliumhydrogencarbonat.

19. 1,3-Bis[[p-(dimethylamino)benzyliden]amino]-2-methylimidazoliumhydrogencarbonat.

20. 1,3-Bis[[p-(Dimethylamino)benzyliden]amino]-2-phenylimidazoliumhydrogencarbonat.

21. Verfahren zur Herstellung von Verbindungen gemäss einem der Ansprüche 1 bis 20, dadurch gekennzeichnet, dass man eine Verbindung der allgemeinen Formel

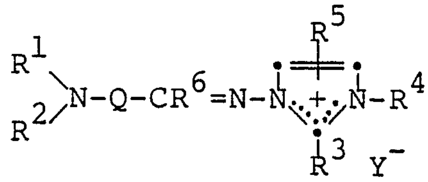

II

worin $R^1$, $R^2$, $R^3$, $R^4$, $R^5$, $R^6$, Q und die gestrichelte Linie obige Bedeutung besitzen, und $Y^-$ ein Anion bedeutet,
mit einer konzentrierten, wässrigen Alkalimetallhydrogencarbonatlösung behandelt.

22. Verfahren gemäss Anspruch 21, dadurch gekennzeichnet, dass man eine gesättigte Hydrogencarbonatlösung verwendet.

23. Verfahren gemäss Anspruch 21 oder 22, dadurch gekennzeichnet, dass man eine Natriumhydrogencarbonatlösung verwendet.

24. Verfahren gemäss einem der Ansprüche 21-23, dadurch gekennzeichnet, dass es in Gegenwart eines organischen Lösungsmittels durchgeführt wird.

25. Verfahren gemäss einem der Ansprüche 21-24, dadurch gekennzeichnet, dass $Y^-$ ein Halogenidanion ist.

26. Verfahren zur Herstellung von Verbindungen der allgemeinen Formel

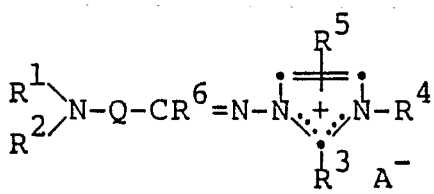

III

worin $R^1$, $R^2$, $R^3$, $R^4$, $R^5$, $R^6$, Q und die gestrichelte Linie obige Bedeutung besitzen, und $A^-$ ein pharmazeutisch annehmbares Anion bedeutet,
dadurch gekennzeichnet, dass man eine Verbindung gemäss Anspruch 1 mit der dem pharmazeutisch annehmbaren Anion $A^-$ entsprechenden Säure AH behandelt.

27. Verwendung von Verbindungen gemäss einem der Ansprüche 1-20 zur Herstellung von diesen Verbindungen entsprechenden pharmazeutisch annehmbaren Salzen.

Patentansprüche für den folgenden Vertragstaat : AT

1. Verfahren zur Herstellung von Verbindungen der allgemeinen Formel

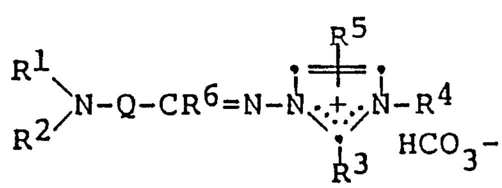

I

worin das Symbol Q Arylen oder Heteroarylen, die Gruppe $-NR^1R^2$ eine basische Aminogruppe, $R^3$ Wasserstoff, niederes Alkyl, niederes Hydroxylalkyl, niederes Alkoxyalkyl, niederes Halogenalkyl, niederes Alkylthio, niederes Alkoxy oder die Gruppe $-(A)_n-Ra$, $R^4$ eine gesättigte oder partiell ungesättigte niedere Kohlenwasserstoffgruppe, eine basische Aminogruppe oder die Gruppe -N=CRc-Rb, -CHRcRd, -NH-CHRcRd, -NH-CO-Re oder -CHRc-CO-Re, $R^5$ Wasserstoff, niederes Alkyl, niederes Hydroxylalkyl, niederes Alkoxyalkyl, niederes Halogenalkyl, Aryl oder einen ankondensierten Benzolring, $R^6$ Wasserstoff oder niederes Alkyl, Ra und Rb je Aryl, Heteroaryl oder eine basische Aminogruppe, Rc Wasserstoff oder niederes Alkyl, Rd Aryl oder Heteroaryl, Re Wasserstoff, eine gegebenenfalls über ein Sauerstoffatom gebundene, gesättigte oder partiell ungesättigte niedere Kohlenwasserstoffgruppe oder eine gegebenenfalls über eine niedere Alkylgruppe gebundene Aryl-, Heteroaryl-oder basische Aminogruppe, A Vinylen oder niederes Alkylen, n die Zahl 0 oder 1 und die gestrichelte Linie eine zusätzliche Doppelbindung bedeuten,

dadurch gekennzeichnet, dass man eine Verbindung der allgemeinen Formel

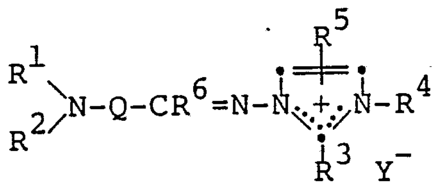

II

worin $R^1$, $R^2$, $R^3$, $R^4$, $R^5$, $R^6$, Q und die gestrichelte Linie obige Bedeutung besitzen, und $Y^-$ ein Anion bedeutet,
mit einer konzentrierten, wässrigen Alkalimetallhydrogencarbonatlösung behandelt.

2. Verfahren gemäss Anspruch 1, dadurch gekennzeichnet, dass man eine gesättigte Hydrogencarbonatlösung verwendet.

3. Verfahren gemäss Anspruch 1 oder 2, dadurch gekennzeichnet, dass man eine Natriumhydrogencarbonatlösung verwendet.

4. Verfahren gemäss einem der Ansprüche 1-3, dadurch gekennzeichnet, dass es in Gegenwart eines organischen Lösungsmittels durchgeführt wird.

5. Verfahren gemäss einem der Ansprüche 1-4, dadurch gekennzeichnet, dass $Y^-$ ein Halogenidanion ist.

6. Verfahren gemäss einem der Ansprüche 1-5, worin $R^3$ niederes Alkyl bedeutet.

7. Verfahren gemäss einem der Ansprüche 1-5, worin $R^3$ die Gruppe $-(A)_n-Ra$ bedeutet.

8. Verfahren gemäss Anspruch 7, worin n die Zahl 0 und Ra eine unsubstituierte oder durch einen, zwei oder drei Substituenten aus der Gruppe bestehend aus niederem Alkyl, niederem Alkoxy, niederem Alkylthio, Halogen, Nitro und Di(niederem Alkyl)amino substituierte Phenylgruppe bedeuten.

9. Verfahren gemäss Anspruch 8, worin Ra eine unsubstituierte oder durch niederes Alkyl, niederes Alkoxy oder Halogen mono-substituierte Phenylgruppe bedeutet.

10. Verfahren gemäss Anspruch 8, worin n die Zahl 1, A Vinylen, Ra die Gruppe -NRR′ und R und R′ je Wasserstoff oder niederes Alkyl oder zusammen mit dem Stickstoffatom einen gegebenenfalls durch eine oder zwei niedere Alkylgruppen substituierten, 5-oder 6-gliedrigen, gesättigten Heterocyclus, der ein oder zwei Stickstoffatome oder ein Stickstoffatom und ein Sauerstoff-oder Schwefelatom als Heteroatom enthält, bedeuten.

11. Verfahren gemäss Anspruch 10, worin R und R′ je niederes Alkyl oder zusammen mit dem Stickstoffatom 4-Morpholinyl oder 1-Piperidinyl bedeuten.

12. Verfahren gemäss einem der Ansprüche 1-11, worin $R^5$ Wasserstoff bedeutet.

13. Verfahren gemäss einem der Ansprüche 1-12, worin $R^4$ die Gruppe $-N=CR^6-Q-NR^1R^2$ bedeutet.

14. Verfahren gemäss einem der Ansprüche 1-12, worin $R^4$ die Gruppe -N = CRc-Rb bedeutet.

15. Verfahren gemäss Anspruch 14, worin Rc Wasserstoff bedeutet.

16. Verfahren gemäss Anspruch 14 oder 15, worin Rb eine unsubstituierte oder durch einen, zwei oder drei Substituenten aus der Gruppe bestehend aus niederem Alkyl, niederem Alkoxy, niederem Alkylthio, Halogen und Nitro substituierte Phenylgruppe bedeutet.

17. Verfahren gemäss einem der Ansprüche 1-16, worin Q 1,4-Phenylen bedeuten, das noch durch einen oder zwei Substituenten aus der Gruppe bestehend aus niederem Alkyl und niederem Alkoxy substituiert sein kann.

18. Verfahren gemäss Anspruch 17, worin Q 1,4-Phenylen bedeutet.

19. Verfahren gemäss einem der Ansprüche 1-18, worin $R^1$ und $R^2$ je niederes Alkyl bedeuten.

20. Verfahren gemäss einem der Ansprüche 1 bis 19, worin $R^6$ Wasserstoff bedeutet.

21. Verfahren gemäss einem der Ansprüche 1-5, dadurch gekennzeichnet, dass man 1,3-Bis[[p-(dimethylamino)benzyliden]amino]-2-[2-(dimethylamino)vinyl]imidazoliumhydrogencarbonat herstellt.

22. Verfahren gemäss einem der Ansprüche 1-5, dadurch gekennzeichnet, dass man 1,3-Bis[[p-(dimethylamino)benzyliden]amino]-2-isopropylimidazoliumhydrogencarbonat herstellt.

23. Verfahren gemäss einem der Ansprüche 1-5, dadurch gekennzeichnet, dass man 1,3-Bis[[p-(dimethylamino)benzyliden]amino]-2-methylimidazoliumhydrogencarbonat herstellt.

24. Verfahren gemäss einem der Ansprüche 1-5, dadurch gekennzeichnet, dass man 1,3-Bis[[p-(Dimethylamino)benzyliden]amino]-2-phenylimidazoliumhydrogencarbonat herstellt.

25. Verfahren zur Herstellung von Verbindungen der allgemeinen Formel

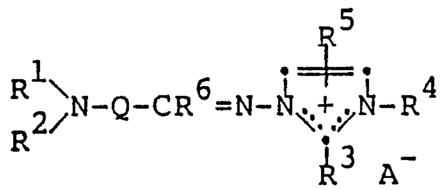

III

worin $R^1$, $R^2$, $R^3$, $R^4$, $R^5$, $R^6$, Q und die gestrichelte Linie die in Anspruch 1 angegebene Bedeutung besitzen, und $A^-$ ein pharmazeutisch annehmbares Anion bedeutet,
dadurch gekennzeichnet, dass man eine Verbindung der in Anspruch 1 definierten Formel I mit der dem pharmazeutisch annehmbaren Anion $A^-$ entsprechenden Säure AH behandelt.

26. Verwendung von Verbindungen der in Anspruch 1 definerten Formel I zur Herstellung von diesen Verbindungen entsprechenden pharmazeutisch annehmbaren Salzen.

Europäisches Patentamt

# EUROPÄISCHER RECHERCHENBERICHT

Nummer der Anmeldung

EP 87 11 3797

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 577 553 (FERLAUTO)<br>--- | | C 07 D 233/56<br>C 07 D 233/84<br>C 07 D 233/70<br>C 07 D 233/22<br>C 07 D 233/20<br>C 07 D 233/38<br>C 07 D 233/42<br>A 61 K 31/415 |
| A | FR-A-2 196 157 (SANDOZ)<br>--- | | |
| P,A | EP-A-0 200 947 (HOFFMANN-LA ROCHE)<br>----- | | |
| | | | **RECHERCHIERTE SACHGEBIETE (Int. Cl.4)** |
| | | | C 07 D 233/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 09-01-1988 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)